# EUROPEAN PATENT SPECIFICATION

(11) **EP 1 985 708 B1**
(45) Date of publication and mention of the grant of the patent: **15.04.2015**
(21) Application number: 07008666.5
(22) Date of filing: 27.04.2007
(51) Int. Cl.: C12N 15/86, C07K 7/06, C07K 7/08, G01N 33/68

(54) **Selective targeting of viruses to neural precursor cells**
Selektives Targeting von Viren in neurale Vorläuferzellen
Ciblage sélectif de virus pour les précurseurs de cellules neurales

(43) Date of publication of application: 29.10.2008
(73) Proprietor: Universität Rostock, 18055 Rostock (DE)
(72) Inventor: Pützer, Brigitte, Prof. Dr. rer. nat., 18057 Rostock (DE)
(74) Representative: UEXKÜLL & STOLBERG

(56) References cited:
- WO-A-99/19500
- WO-A-2006/054262
- WO-A-2006/105392
- DATABASE UniProt [Online] 1 March 2003 (2003-03-01), "Adult male epididymis cDNA, RIKEN full-length enriched library, clone:9230102G06 product:hypothetical protein, full insert sequence." XP002447976 retrieved from EBI accession no. UNIPROT:Q8C5A9 Database accession no. Q8C5A9
- DATABASE UniProt [Online] 24 January 2006 (2006-01-24), "Predicted protein." XP002447977 retrieved from EBI accession no. UNIPROT:Q2UE98 Database accession no. Q2UE98
- BÖCKMANN M ET AL: "Discovery of targeting peptides for selective therapy of medullary thyroid carcinoma." THE JOURNAL OF GENE MEDICINE FEB 2005, vol. 7, no. 2, February 2005 (2005-02), pages 179-188, XP002447952 ISSN: 1099-498X
- SCHMIDT A ET AL: "Analysis of adenovirus gene transfer into adult neural stem cells" VIRUS RESEARCH, AMSTERDAM, NL, vol. 114, no. 1-2, December 2005 (2005-12), pages 45-53, XP005152513 ISSN: 0168-1702
- SCHMIDT A ET AL: "Selective targeting of adenoviral vectors to neural precursor cells in the hippocampus of adult mice: new prospects for in situ gene therapy" STEM CELLS, [Online] 19 July 2007 (2007-07-19), XP009088489 Retrieved from the Internet: URL:http://stemcells.alphamedpress.org/cgi /reprint/2007-0238v1> [retrieved on 2007-08-23]

## Description

Neural precursor cells (NPC) are found throughout the entire adult brain but they generate neurons only in two neurogenic regions: the subgranular zone (SGZ) in the hippocampal dentate gyrus and the lateral wall of the lateral ventricles, the subventricular zone (SVZ). They are attracted to brain lesions, such as areas of neurodegeneration, ischemia and cancer. The properties of NPC in the adult brain make them potentially suitable for restorative cell replacement strategies as well as delivery vehicles for gene therapy. For example they have shown tropism for gliomas and degenerating spinal cord motor neurons in amyotrophic lateral sclerosis transgenic mice. Failing adult hippocampal neurogenesis has been brought into connection with the pathogenesis of disorders as diverse as dementias, major depression, and temporal lobe epilepsy.

The potential for the treatment of CNS disorders, including those affecting the hippocampus has tremendously advanced with the ability to identify specific genes whose defect or absence is responsible for the particular pathological condition, and genes that control precursor cell differentiation. Successful delivery of these therapeutic genes to precursor cells could provide a significant advancement in therapy for certain brain disorders. So far, experimental cell therapy for CNS disorders has been based on the transplantation of *in vitro* expanded and genetically engineered NPC.

However, the normal course of NPC development and migration *in vivo* is controlled by the microenvironment in the neurogenic regions of the brain. Because culture conditions strongly influence the phenotype of cells, culture could markedly alter the cell's response to their environment when reintroduced *in vivo*.

Genes could be introduced into NPC using lentiviral vectors. A number of concerns are however, associated with the use of lentiviruses, as lentiviruses have caused tumors in human patients, due to the fact that the virus is integrated into the genome of the patient.

Different approaches were developed to target the viral vector to a specific cell type. For example adenoviruses were linked to bi-specific monoclonal antibodies which specifically bound to pulmonary endothelium. In another approach, sequences of the epidermal growth factor were fused to a viral protein and the viruses were thus targeted to growth factor receptor positive cells.

WO 2006/054262 discloses that a phage display system can be used to identify peptides which specifically bind receptors on the membrane of neuronal cells.

However, until now it has not been possible to target a respective virus or viral vector to NPC. As a consequence, there thus is still a need for a safe and efficient system for delivering genes selectively to neural precursor cells.

This problem is now solved by a virus particle comprising a DNA sequence and at least one capsid protein, characterized in that the capsid protein is linked to a peptide selectively binding to neural precursor cells, wherein the viral vector is derived from a lentivirus, a retrovirus, an adenovirus, an adeno-associated virus, an Epstein-Barr virus, a herpes virus, an attenuated HIV or a vaccinia virus, and the neural precursor cells have the ability to self-renew and express the cell marker nestin, further characterized in that the peptide which selectively binds to neural precursor cells comprises at least one of the following amino acid sequences TPIQDYT (SEQ ID NO:1), QTRFLLH (SEQ ID NO:2), VPTQSSG (SEQ ID NO:3) or QTFTRMY (SEQ ID NO:4).

In the present application cells are considered to represent neural precursor cells, if they have the ability to proliferate and express the cell marker nestin.

Neural precursor cells or NPC are optionally further characterized by the expression of the cell markers Musashi, Sox1/2 and/or GFAP and may have the ability to differentiate into neuronal and glial cell lines.

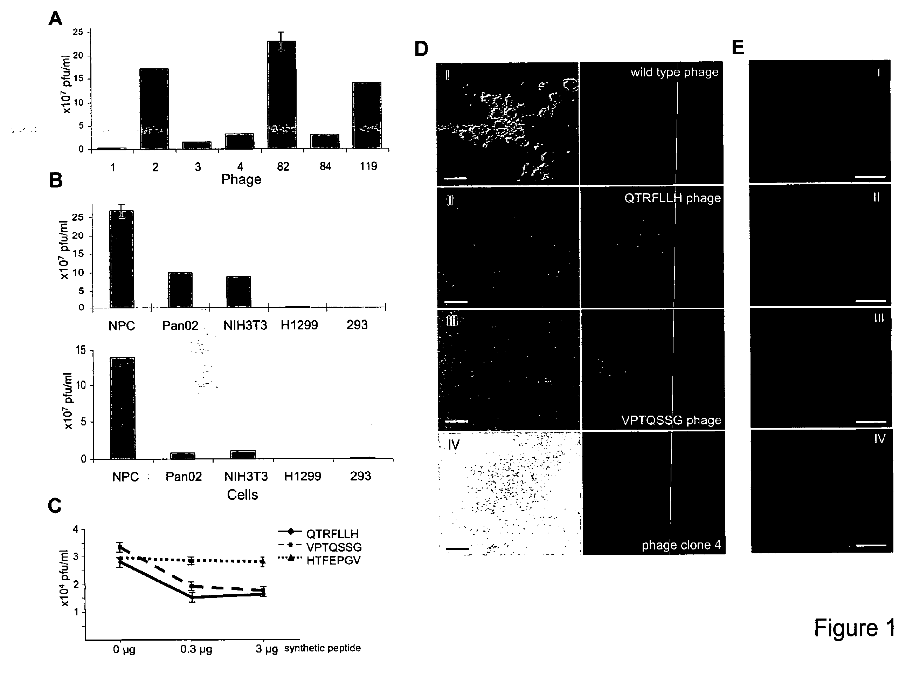

Surprisingly, the present inventors were able to identify peptides selectively binding to neural precursor cells. In accordance with the present invention, a peptide is considered to bind selectively to neural precursor cells, if the binding of a phage expressing the peptide or the binding of a virus comprising the peptide linked to a virus capsid protein or integrated into the virus capsid to neural precursor cells is reduced by a factor of at least 25%, preferably by at least 35% or by about 50%, by a peptide having the same sequence if present in the same concentration under conditions, where a peptide of unrelated sequence reduces the binding of the selectively binding peptide by less than 10%. An unrelated peptide differs in essentially all residues from the amino acid sequence of the peptide selectively binding to NPC. The assay thus analyzes human or murine NPC binding competition between the peptide in isolated form on the one hand and the same peptide expressed by a phage or linked to a capsid protein of a virus on the other hand. Respective assays are illustrated in Example 2 and illustrative results are shown in Figure 1c.

In the context of the present invention the peptides selectively binding to NPC are optionally further characterized as binding differentiated neural cells with less than 50%, preferably less than 75% or less than 85% of the affinity of binding NPC. Binding affinity of the peptides can be determined for example by incubating phages expressing the peptide on their surface with the respective cell line, washing off unbound material and subsequently washing off bound phages and determining the number of the bound phages. Respective assays, which use other cells than differentiated neurons, are illustrated in Example 3 and in Figure 1B.

As shown for the first time in the present application, these peptides allow targeted delivery of genes present as part of the genome of a viral particle to neural precursor cells, where viruses are internalized and the genes can be expressed.

The virus particle comprises DNA sequences of a viral vector, which vector is derived from a lentivirus, a retrovirus, an adenovirus, an adeno-associated virus, an Epstein-Barr virus, a herpes virus, an attenuated HIV or a vaccinia virus. The use of adenovirus vector sequences for the preparation of the viral particles of the present invention is particularly preferred.

The peptide which binds selectively to neural precursor cells can be linked to the virus particle in any manner that provides a sufficient bonding to allow the viral particle to be selectively delivered to NPC. The peptide which selectively binds to neural precursor cells may be covalently linked to a capsid protein of the virus or the DNA sequence encoding the peptide may be genetically integrated into the DNA expressing the virus capsid protein. In the latter alternative the peptide selectively binding to NPC is expressed as part of the capsid protein, i.e. as a fusion protein of the peptide which binds selectively to neural precursor cells and the capsid protein. In this embodiment the DNA sequence encoding the peptide selectively binding to NPC may replace a sequence of the capsid protein or may be included into the sequence. It is especially preferred that the peptide replaces that part of the sequence of the virus capsid protein which mediates binding of the wild type virus to its host cells.

According to one embodiment, the peptide which selectively binds to neural precursor cells consists of 5 to 15 amino acids, preferably 5 to 10 amino acids, wherein a length of 7 amino acids is specifically preferred.

The peptide which selectively binds to neural precursor cells comprises at least one of the following amino acid sequences TPIQDYT (SEQ ID NO:1), QTRFLLH (SEQ ID NO:2), VPTQSSG (SEQ ID NO:3) or QTFTRMY (SEQ ID NO:4). Preferably, the peptide consists of one of these sequences. The virus particle further comprises a DNA sequence.

The virus particle may comprise a replication-defective vector, i.e. a vector which is not capable of generating additional copies of the DNA sequence contained in the viral particle. Alternatively, the viral particle may comprise a self-replicating but not infectious virus or even a fully infectious virus. the virus vector may be a replication-competent viral vector or a high-capacity (helper-dependent) vector (third generation). the viral particle may comprise a replication-defective adenoviral vector, i.e. DNA sequences derived from the genome of an adenovirus, which no longer have the ability to generate an infectious viral particle, but which may still associate with one or several capsid proteins to a viral particle.

The virus particle preferably comprises a DNA sequence comprising a gene encoding a therapeutic protein or a sequence encoding a small interfering RNA (siRNA). A large number of therapeutic proteins can be expressed by the respective genes. The virus may for example comprise a DNA sequence which comprises a gene encoding a protein which promotes lineage-specific differentiation of neural precursor cells, such as growth factors (for example EGF or FGF), neurotrophic factors (such as BDNF, neurotrophin NT-3, NT-4, CTNF, GDNF, leukemia inhibitory factor LIF, cardiotrophin-1 as well as oncostatin M etc).

Alternatively or additionally, the virus particle may contain a DNA sequence comprising a gene encoding a therapeutic protein, such as the Neprilysin gene, the GDNF sequence, glutamic acid decarboxylase GAD-65/GAD-67 or the adenosine kinase inhibitor sequence. The DNA sequence may further encode such as siRNAs against BACE1, apolipoprotein E (ApoE) or GDNF.

According to a further aspect, the present disclosure relates to a viral vector, comprising a DNA sequence encoding fusion polypeptide of a viral capsid protein and a peptide selectively binding to neural precursor cells as described above. The viral vector preferably is an adenoviral vector comprising a DNA sequence encoding a therapeutic protein or a sequence encoding a siRNA.

DNA sequences, such as viral vectors are provided, which comprise a DNA sequence encoding fusion polypeptide of a viral capsid protein and a peptide selectively binding to neural precursor cells as described above.

The present invention further provides proteins and peptides which selectively bind to NPC. The peptides comprise the amino acid sequence TPIQDYT (SEQ ID NO:1), QTRFLLH (SEQ ID NO:2), VPTQSSG (SEQ ID NO:3) or QTFTRMY (SEQ ID NO:4). The peptides may be present in isolated form, as part of a fusion protein, chemically linked or cross-linked to a protein, such as a virus capsid protein, or expressed as part of the virus capsid/fiber protein. In a preferred embodiment the peptide selectively binding to NPC consists of the the amino acid sequence TPIQDYT (SEQ ID NO:1), QTRFLLH (SEQ ID NO:2), VPTQSSG (SEQ ID NO:3) or QTFTRMY (SEQ ID NO:4).

In a further aspect the present invention relates to a pharmaceutical composition comprising a virus particle or a viral vector as characterized above. The pharmaceutical composition may further comprise a pharmaceutically acceptable carrier, such as a phosphate-buffered saline solution (PBS): 0.02 M sodium phosphate buffer with 0.15 M sodium chloride, pH adjusted to 7.4.

The present invention further provides the use of these virus particles for the preparation of a medicament for treating a neurodegenerative disease, preferably in human patients. The virus particles of the present invention enable new treatment approaches for neurodegenerative disease, such as Alzheimer Disease or Parkinson Disease, ischemia, dementia, depression, temporal lobe epilepsy, Multiple Sclerosis (MS), Amyotrophic Lateral Sclerosis (ALS) or cancer.

In a further aspect the present disclosure relates to a method of identifying a peptide selectively binding to neural precursor cells comprising incubating a culture of early stage neurospheres with a peptide phage library and recovering enriched phages.

In the context of the present invention a cell culture is referred to as early stage neurospheres, if the cells have not been maintained in culture for more than 12 weeks, preferably not more than 5 weeks and most preferably not more than 10 days, and the cells comprise free floating cell clusters of at least 100 cells, preferably between 100 and 10.000 cells, which comprise proliferative cells that express the cell marker nestin. The cell clusters preferably comprise cells, which further express Musashi, Sox1/2, GFAP and/or PSA-NCAM. Only a small proportion of early stage neurospheres (up to 10%) shows in vitro stem cell properties. The shorter the cells have been maintained in cell culture, the higher the amount of cells with stem cell properties will be.

The present invention surprisingly shows that peptides of a phage display library can be used to identify peptides having a high selective affinity for neural precursor cells. In the subsequent examples murine neurospheres were used. High affinity of the peptides for human and murine NPC was confirmed. It was also shown that the peptides thus obtained do not have a high affinity for murine or human cells other than NPC, such as tumor cells or fibroblasts.

In the methods of identifying a peptide selectively binding to neural precursor cells as described above one could evidently also use human neurospheres or human cell lines or tissues comprising human neural precursor.

The virus particles as described above can be obtained by a method of generating a virus particle, which method comprises the identification of a peptide selectively binding to neural precursor cells using phage library screening of a culture of early stage neurospheres, recovering enriched phages and linking the peptide thus identified to a virus particle or expressing the peptide as part of the virus capsid/fiber protein by genetic integration of the peptide encoding DNA sequence.

The peptide selectively binding to neural precursor cells can be linked to the viral capsid protein in various ways. For example, the peptide can be chemically linked to the viral capsid protein using polyethylene glycol. Alternatively, one may genetically integrate the DNA sequence encoding peptide into the virus capsid, or generate a fusion protein comprising the peptide and the capsid protein, by fusing the DNA sequences encoding the peptide and the protein and recombinant expression of that DNA sequence.

The present invention also provides pharmaceutical compositions comprising a virus particle or a viral vector as described above.

### Brief Description of the Figures:

**Figure 1**: Neural precursor cell specificity of pre-selected phages in vitro. (a) The binding efficiency of phages recovered after a third round of biopanning on cultured neurospheres was tested by titration assay. Precursor cells isolated from adult mouse brains were incubated with the indicated phage clones including the best binding phages 2, 82, and 119 compared to a representative choice of lower binding phages (1, 3, 4, 84) for 4 h. The phage titer was determined after incubation of eluted phages with Escherichia coli (ER2738) by counting plaque forming units (pfu). Bars show standard error of the mean (s.e.m.) from plating in duplicate. (b) Titration results of phage clones 82 (QTRFLLH, SEQ ID NO:2, upper panel) and 119 (VPTQSSG, SEQ ID NO:3, bottom panel) after binding to neurospheres and different other murine (PanO2, NIH 3T3) or human (H1299, 293) cell lines. (c) Effect of synthetic peptides on phage binding to adult neural precursor cells. Neurospheres were incubated with phages in the absence or presence of indicated concentrations of specific QTRFLLH (SEQ ID NO:2) or VPTQSSG (SEQ ID NO:3) peptides for 1 h. Unspecific HTFEPGV (SEQ ID NO:5) peptide was used as a control. The binding efficiency was determined by titration. (d) Immunofluorescent microscopy of cultured neurospheres incubated with the wild-type (I), QTRFLLH (II), SEQ ID NO:2, VPTQSSG (III), SEQ ID NO:3, or unspecific control phage 4 (IV) for 24 h. Phage binding and internalization was visualized using mAb against M13. Scale bar, 20 µm (panel II, 200 µm). (e) Immunofluorescent microscopy of neurospheres infected with PEGylated AdRFP.QTRFLLH (I, II) or AdRFP.VPTQSSG (III, IV) in the absence (I, III) or presence of 3 µg/ml specific synthetic peptide (II, IV). RFP expression was detected at 4 days after infection, Scale bar, 200 µm.

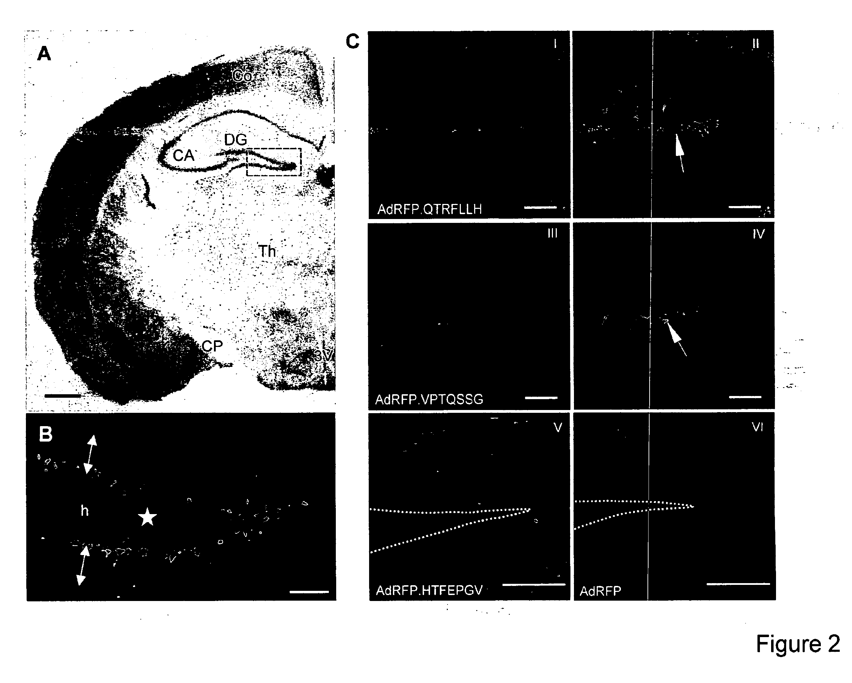

**Figure 2**: In vivo neural precursor cell targeting capability of the QTRFLLH and VPTQSSG peptides linked to adenovirus in adult mice. (a) Nissl stained coronal section of a pNestin-GFP transgenic mouse brain hemisphere through the region of the dorsal hippocampus containing the cornu ammonis (CA) and the dentate gyrus (DG). Further principle structures as cortex (Co), the cerebral peduncle (CP), thalamus (Th) and the third ventricle (3V) are indicated. The outlined area of the DG containing the population of NPC is shown in detail in Fig. 2b. (b) Parallel section through the dentate gyrus clearly shows that the nestin-containing somata of the GFP-expressing neural precursor cells are confined to the subgranular zone located between the hilus (h) and the granule cell layer (arrows). The radial glia like morphology of the so called type-1 cells is obvious (light gray). The site of Ad-virus injection for in vivo stem cell targeting is indicated by a star. (c) Fluorescence-activated laser scanning microscopy of the adult DG from pNestin-GFP transgenic mice after injection of AdRFP.QTRFLLH (I) and AdRFP.VPTQSSG (III). In both cases high numbers of transduced cells with a type-1 cell morphology can be detected (gray cells). In the images merged for RFP and GFP expression in nestin-containing precursor cells the co-localization is obvious (double stained cells are indicated by arrows) (II, IV). As controls AdRFP.HTFEPGV with an unspecific peptide (V) and AdRFP (wild-type capsid, VI) were used and in both cases a lack of transduced neural precursor cells was observed in the DG. The border between the hilus and the granular zone is depicted by the dotted line. Scale bars, 500 µm (a), 100 µm (b, c).

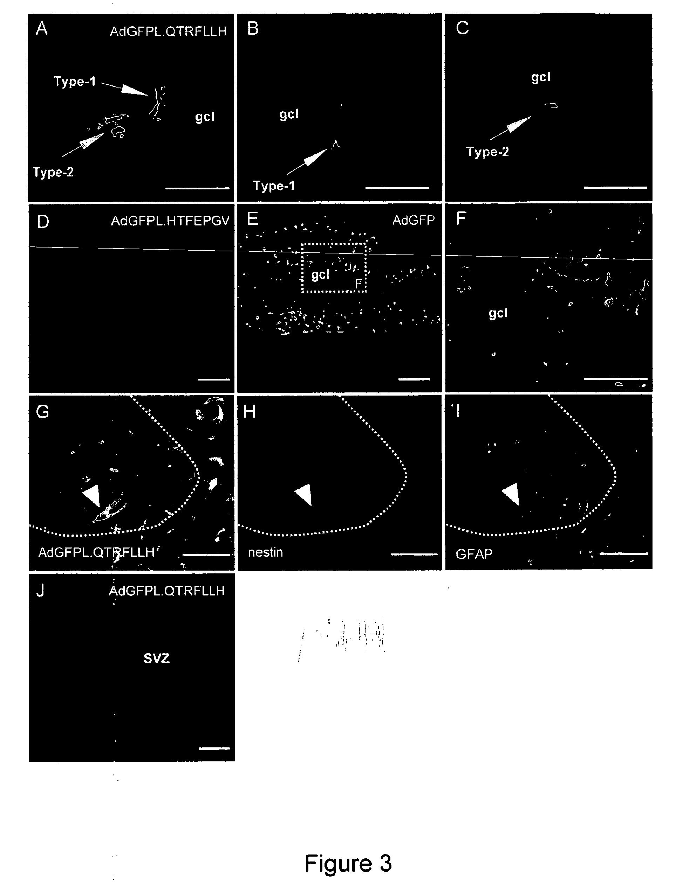

**Figure 3**: Selective transduction of neural precursor cells in the adult C57BL/6 mouse brain by a capsid mutated AdGFPL.QTRFLLH vector. PEGylated AdGFPL.QTRFLLH (a-c), AdGFPL.HTFEPGV (d) or AdGFP (e, f) were injected into the DG of adult C57BL/6 mice. (a-c) AdGFLP.QTRFLLH infected GFP-expressing (gray cell body) type-1 or type-2 precursor cells are indicated by arrows. (a, b) The radial glia-like morphology of the type-1 cells and the location of their somata in the subgranular zone is shown. (d) AdGFPL.HTFEPGV failed to transduce cells in the dentate gyrus. (e, f) Cells with a neuronal or a mature astrocyte morphology were infected by AdGFP. (g-i) Immunohistochemical counterstaining of AdGFPL.QTRFLLH transduced NPC for GFAP and nestin. A GFP expressing type-1 cell (arrowhead, g) immunoreactive for nestin (arrowhead, h) and GFAP (arrowhead, i) is marked by an arrowhead. The border between the subgranular and the granular zone is depicted by the dotted line. j) GFP-positive cells were not detected after injection of AdGFPL.QTRFLLH in the SVZ. Scale bars, 100 µm (a-f, j), 40 µm (g-i).

### Example 1: Materials and Methods

### Cell culture and peptides:

Neural precursor cells were isolated from adult C57BL/6 hippocampus (Charles River Lab., USA) and expanded as previously described¹. 293, H1299, Pan 02, NIH 3T3 cell lines were obtained from ATCC. Cell cultures were maintained in Dulbeccos's modified Eagle's medium (DMEM, Life Technologies) supplemented with 10% fetal calf serum (FCS, Biochrom) and 1% penicillin G/streptomycin (Life Technologies) in 5% CO₂ at 37°C. All synthetic peptides were synthesized and purified by high-performance liquid chromatography (HPLC) (Metabion).

### Phage Display:

A Ph.D.-7 Phage Display Library (New England Biolabs) was amplified, purified and titered according to the manufacturer's protocol. Briefly, neural precursor cells were incubated with 1.5 x 10¹¹ plaque forming units (pfu) of input phage library for 4 h at 4°C. Phage recovery was performed using biopanning and rapid analysis of selective interactive ligands (termed BRASIL) optimization method. The panning procedure was repeated 3 times. Single phage plaques were picked in a last step and analyzed. **Titration assay**. Phage binding of infected cells *in vitro* was quantified as described² Recovered phages were incubated with competent bacteria (*Escherichia coli* ER2738) and phage titer was determined by counting pfu.

### Immunofluorescence and Laser Scanning Microscopy:

Cells were seeded on glass coverslips and fixed with 10% formaldehyde/PBS. After incubation with 1.5 x 10¹¹ pfu of selected phages for 4-8 h at room temperature slides were washed and treated with 500 mM NaCl/50 mM glycine to remove weakly bound phages. Cells were permeabilized with PBS/0.2% Triton X-100 and blocked with PBS/1% BSA containing 10% FCS. Slides were washed twice with PBS/1% BSA and incubated with anti-M13 antibody (Amersham) for 1 h. After washing with PBS the slides were incubated with appropriate secondary AlexaFluor⁵⁴⁶-conjugated anti-mouse antibody (Molecular Probes) and subjected to fluorescence-activated laser scanning microscopy.

**Competition Assays**. Phages displaying QTRFLLH, VPTQSSG or HTFEPGV (SEQ ID NO:5) motifs were titrated and 1 x 10⁹ pfu/ml were incubated with 1 x 10⁶ cells either alone or together with QTRFLLH (SEQ ID NO:2), VPTQSSG (SEQ ID NO:3) or HTFEPGV (SEQ ID NO:5) synthetic peptide for 1h. Cells were washed five times with PBS containing 1% BSA and 0.1% Tween-20. Phages were eluted by freeze-cut technique and pelleted by centrifugation. The amount of bound phages was determined by counting pfu after bacterial infection. For analysis of peptide-mediated specific AdRFP.QTRFLLH or AdRFP.VPTQSSG binding, 5 x 10⁵ adult NPC were infected with 10⁷ plaque-forming units (pfu) of PEGylated virus in the absence or presence of synthetic peptide (0.3-3.0 µg/ml). Virus-cell interaction was visualized by immunofluorescence and laser scanning microscopy.

### Adenovirus Vector Production and Peptides:

Adenovirus serotype 5-derived vectors AdGFP (wild-type capsid) and pAdTLY477RAE (with GFP-Luc and therefore named AdGFPL, Fiber mutant, CAR-binding ablated) viruses have been described earlier^{3, 4}. For generation of the recombinant Ad vector that expresses red fluorescent protein (Ad-RFP), RFP cDNA from the pDsRed Monomer plasmid (Clontech) was inserted into the KpnI/NotI restriction sites of the pShuttleCMV (AdEasy system). Virus was generated by homologous recombination following cotransfection with pAdEasyl in *E. coli* BJ5183. All viruses were propagated, purified and titrated by standard methods. Synthetic peptides were covalently linked to Ad vectors by polyethylene glycol (PEG, Shearwater Polymers). Briefly, bifunctional PEG was added to the virus with 5% w/v. To allow coupling of PEG to the adenovirus surface, samples were incubated by stirring for 1h at room temperature. Repeated CsCl gradient centrifugation was performed to separate unbound PEG from the PEGylated virus followed by dialysis overnight at 4°C. Peptides dissolved in PBS containing 5% sucrose to a final concentration of 10 mM were added to the PEGylated virus at a concentration of 1 mM and incubated by stirring for 4 h at 4°C. Separation of unreacted peptide was achieved by dialysis overnight.

### Mice Experiments:

Young adult male C57BL/6 mice were obtained from Charles River Laboratories. Transgenic mice that express GFP under the control of the rat nestin gene regulatory region (referred to as pNestin-GFP mice) were generated by M. Yamaguchi⁵ (Department of Physiology, University of Tokyo, Japan). Mice were housed in a dedicated pathogen-free animal facility under a 12 h light/dark cycle with free access to food and water. All animal procedures were conducted in accordance with the german guidelines and with approval of the local authorities. Adenoviral vectors (10⁵ to 10⁶ pfu/mouse) were injected into one of the regions containing NPC (subventricular zone = SVZ, lateral ventricle = LV, dentate gyrus of the hippocampus = GD) of the adult C57BL/6 and pNestin-GFP mouse brain, respectively. Briefly, mice were anesthetized by intraperitoneal injection of Ketamin (75 mg/kg) and Rompun (5.8 mg/kg) and mounted in a mouse adaptor (Stoelting Co) fixed in a rat stereotaxic apparatus (Kopf). The skull was opened with a dental thrill and Ad vectors were injected into the SVZ (2 µl, AP: +1.2 mm, L: -0.8 mm, DV: -2.4 mm (D), coordinates according to bregma⁶), the LV (3µl, AP: ±0 mm, L: -0.8 mm, DV : -1.8 mm (D)) or the GD (2µl, AP: -1.9 mm, L: -1.2 mm, DV: -1.8 mm (D)) by using a glass capillary with an outer diameter of about 70 µm mounted onto a 5 µl Hamilton syringe.

### Immunohistochemistry:

After anaesthesia with an overdose of pentobarbital-Na⁺ (45 mg/kg), 3 days after virus-injection (n = 3 - 5 per group), mice were perfused with 5 ml isotonic NaCl-solution, followed by 50 ml of 3.7% paraformaldehyd (dissolved in 0.1 M PBS, pH 7.4). Brains were immediately removed from the skull, postfixed overnight, followed by an incubation overnight in PBS containing 20% sucrose at 4°C and finally frozen in isopentane (-50°C) and stored at -8.0°C. Brains were cut in 30 µm thick sections using a cryostat. Sections were washed three times with PBS, blocked for 1 h at room temperature (PBS with 3% BSA, 3% normal goat serum and 0,05% Triton X-100), and incubated with primary antibodies (Nestin, 1:100, ms, StemCell; GFAP, 1:400, rb, Sigma) in PBS/1% BSA/0,025% Triton X-100 overnight at 4°C. After washing, the sections were incubated with Cy2 or Cy3-conjugated anti-mouse (Dianova) and anti-rabbit AMCA (1:100 Dianova) secondary antibodies. Brain sections were mounted onto SuperFrostPlus coated glass slides, air dried, covered with a fluorescence mounting medium and subjected to fluorescence-activated laser scanning microscopy.

### Example 2: Identification of Peptides selectively binding NPC

To identfy peptide ligands that selectively bind NPC from adult mouse hippocampus, the phage display technology was used. Neural precursor cells in vitro were obtained using methods known in the art and their ability to proliferate and to differentiate into glia and neurons was confirmed. Cultures of primary neurospheres established from the hippocampal area of adult C57BL/6 mice were incubated with a 7mer phage library that offers the possibility to identify small peptide ligands. Neurospheres contain only few true stem cells and show differentiation at the core, making them a heterogeneous cluster of cells¹⁶. To increase the number of stem cells early stage neurospheres were used. In these cultures a large proportion of the cells is proliferative and expresses precursor cell marker nestin. Moreover, a two-stage experiment was designed:
First cultured cells were used to identify peptides with presumed high specificity. Then these peptides were tested in vivo to confirm their sensitivity with immunhistological tools and according to the criteria developed to identify precursor cells in the neurogenic regions of the adult hippocampus.

Biopanning was repeated three times to enrich the pool of phages in favour of the thightest binding sequence, resulting in more than 130 putative ligands after the third round. These candidates were pre-selected by comparing their binding efficiency for nestin-positive neurospheres *in vitro* using a titration assay (**Fig. 1a**). Phage clones 2, 82, and 119 demonstrated the strongest binding to NPC (10- to 20-fold stronger) compared to all others.

Peptide sequences from the selected phages 2, 82 (TPIQDYT, QTRFLLH), and 119 (VPTQSSG) were found 2 to 3 times, respectively. One sequence, QTFTRMY, appeared four times. Other peptides formed clusters depending on their motifs, for example QT, SS, SL, and PL.

### Example 3: Confirmation of Binding Specificity by Screening Affinity in vitro

To further determine the specificity of the identified phages 2, 82, and 119 for NPC, their binding efficiency on additional cell lines of murine (Pan 02, NIH 3T3) and human (H1299, 293) origin was examined. Among these, phages containing the sequence QTRFLLH (**Fig. 1b****, upper panel**) and VPTQSSG (**Fig. 1b****,** bottom) exhibited the strongest binding activity to cultured NPC compared to other cell lines. Phage-cell interaction of the best binding QTRFLLH phage to NPC was 2.5 times stronger than to Pan 02 pancreatic cancer cells (1x10⁸ pfu/ml) and to NIH 3T3 fibroblasts (9x10⁷ pfu/ml), 83 times stronger than to H1299 lung cancer cells (3x10⁶ pfu/ml), and 1250 times stronger than to 293 human embryonic kidney cells (2x10⁵ pfu/ml).

Although the affinity of the QTRFLLH phage to bind precursor cells was found to be higher, the VPTQSSG phage bound them more selectively (binding to non-NPC cells was up to 10 times lower; **Fig. 1b****, bottom**). The binding of both phages to NPC was dose-dependently inhibited in, the presence of synthetic peptides, indicating that phage-precursor cell interaction strictly relied on the displayed peptide. A 50% reduction of plaque-forming units was measured by addition of 0.3 ug of synthetic peptides compared to an unspecific control peptide (**Fig. 1c**). Peptide-mediated internalization of a potential gene delivery system is a prerequisite for efficient transgene expression in the target cells. To test both peptides for their capability to communicate this cellular uptake, neurospheres were seeded on glass coverslips and exposed to 1.5 x 10¹¹ pfu/ml purified QTRFLLH or VPTQSSG phage for 8 h. Phages released into the cell upon internalization were detected with anti-M13 antibody that binds to the phage coat protein. Both QTRFLLH and VPTQSSG peptides induced a strong uptake by NPC as indicated by their M13 antibody reactivity (**Fig. 1d**). Infection of cells with the most commonly employed adenovirus serotype 5 is mediated via two cell surface receptors: the fiber protein binds first to the coxsackie-adenovirus receptor (CAR) and virus internalization is then mediated through an interaction between an arginine-glycine-aspartate (RGD) sequence on the adenovirus penton base protein and cell surface ανß3 and ανß5 integrins.

However, previously a complete lack of CAR and av integrin expression on adult NPC, indicating their resistance to adenoviral infection had previously been reported. To determine whether the QTRFLLH and VPTQSSG peptides can mediate virus binding and internalization by NPC *in vitro,* both peptides were covalently linked to an Ad vector (wild-type capsid) that expresses the red fluorescent protein (RFP) and examined their binding specificity in infected NPC after adding 3.0 µg/ml synthetic QTRFLLH or VPTQSSG peptide (**Fig. 1e**). Unaltered RFP expression of AdRFP.QTRFLLH and AdRFP.VPTQSSG infected neurospheres was observed by immunofluorescent microscopy in the presence of unspecific peptide, but could not be' detected in combination with the corresponding specific synthetic peptides. This supports the notion that the new peptid ligands are similarly efficient in mediating adenovirus binding and infection of precursor cells in the adult mouse brain.

### Example 4: In vivo Testing of Peptides

Recombinant adenoviral vectors have been reported most efficient for delivering genes into non-dividing cells such as glial cells and neurons. A critical assumption for utilizing the peptide-targeted Ad vector as a gene delivery system for endogenous NPC in the brain is, however, that it will not transduce other CNS cells. To test the targeting potential of the identified peptides under *in vivo* conditions, the viruses AdRFP.QTRFLLH and AdRFP.VPTQSSG were injected into the hippocampal area of young adult pNestin-GFP transgenic mice (**Fig. 2a**) that express green fluorescent protein (GFP) under the control of the nestin gene regulatory region shown to be essential for nestin expression in NPC¹. In adult mice, GFP fluorescence was observed in regions where endogenous nestin is expressed and neurogenesis continues throughout life such as subventricular zone (SVZ), rostral migratory stream, olfactory bulb and dentate gyrus (DG, **Fig. 2b**). Fluorescence-activated laser scanning microscopy of brain sections from animals receiving AdRFP vectors that display the precursor cell targeting peptides revealed a strong specific labeling of GFP expressing cells in the SGZ (**Fig. 2c****, panel I/II and III/IV**). By contrast, RFP-postive cells were not detectable in the respective region of the adult dentate gyrus after injection of the AdRFP.HTFEPGV virus carrying an unspecific control peptide (**Fig. 2c****, panel V**). A larger spectrum of CNS cells especially with a neuronal and astroglial morphology, but no NPC were transduced by the wild-type AdRFP vector via CAR-binding (**Fig. 2c****, panel VI**). Additionally, the NPC-specific binding properties of the QTRFLLH and VPTQSSG peptides was examined in context of an adenovirus fiber mutant with completely ablated native tropism. For this purpose, the peptides were covalently linked to the capsid-mutated adenovirus AdGFPL by bifunctional TMPEG. PEGylated AdGFPL.QTRFLLH (and AdGFPL.VPTQSSG) or AdGFPL.HTFEPGV, or AdGFP, the vector that contains a wild-type capsid, was subsequently injected into DG, SVZ and lateral ventricle (LV) of adult C57BL/6 mice. Brain sections were subjected to fluorescence-activated laser scanning microscopy. The mutation of both the CAR-binding domain of the fiber and the integrin-binding motif within the penton base in AdGFPL.QTRFLLH (shown representative for both selective adenoviruses) resulted in a specific staining of NPC type-1 and type-2 in the granule cell layer. A detailed inspection of single AdGFPL.QTRFLLH transduced type-1 precursor cells at a higher magnification shows their radial glia-like morphology (**Fig. 3a****-b**). Moreover, type-2 cells known as transiently amplifying precursor cells, which develop from type-1 cells, are located in the subgranular zone (**Fig. 3a****, c**). Conversely, GFP expression was either not detectable by an Ad vector with the unspecific control peptide (**Fig. 3d**) or much wider after injection of the wild-type capsid AdGFP virus transducing several non-NPC cell types such as astrocytes and neurons in the adult GD (**Fig. 3e****, f**). AdGFPL.QTRFLLH transduced cells were identified as nestin and GFAP positive by immunostaining of the hilus and granule cell layer from virus infected brain sections (**Fig. 3g****-i**). This is consistent with an earlier observation that hippocampal precursor cells show glial features. Importantly, however, GFP expression by AdGFPL. QTRFLLH was restricted to precursor cells located in dentate gyrus, and was not detected in other injected brain regions such as the SVZ and the ependyma of the lateral ventricle (**Fig. 3j**).

In summary, the present application shows that it is possible to target antigens on NPC by a genetically modified viral vector linked to the antigen binding peptide. Phage display was carried out using endogenous neurospheres isolated from the hippocampus of adult mice to identify peptides that selectively bind NPC. Thereby, two peptide ligands were identified that mediated good phage binding and internalization to cultured neurospheres.

It was demonstrated that the identified peptides are useful in targeting virus-mediated gene delivery to NPC *in vivo* using an Ad vector with the wild-type capsid and a CAR-binding ablated virus in normal and pNestin-GFP transgenic mice. Analysis of Ad vector distribution after virus injection into the adult hippocampus, known to home precursor cell populations, demonstrated a highly specific infection of type-1 and type-2 precursor cells in the granule cell layer of the hippocampal dentate gyrus by the capsid-mutated Ad vector displaying the specific peptides. Apart from the notable target cell specificity of AdGFPL.QTRFLLH, GFP expression in precursor cells was confined to dentate gyrus, whereas other injected regions such as the subventricular zone and the ependyma of the lateral ventricle were negative. These data show that intrinsic differences in precursor cells between different brain regions can translate into a diverse spectrum of cell-surface receptors on these cells. Consequently, these findings support the conclusion that NPC-specific Ad vectors can be prepared for specific brain regions. The results further support the use of virus vector systems, preferably adenovirus derived vector systems, to specifically target adult NPC as a perfect tool to efficiently manipulate these cells either by direct injection in the brain or systemic vector application. This allows to use the NPC as a source for cell replacement and the virus vectors as delivery systems for therapeutic genes in order to treat various CNS disorders.

### REFERENCES

1. Schmidt, A., Bockmann, M., Stoll, A., Racek, T. & Putzer, B.M. Analysis of adenovirus gene transfer into adult neural stem cells. Virus Res 114, 45-53 (2005).
2. Bockmann, M., Drosten, M. & Putzer, B.M. Discovery of targeting peptides for selective therapy of medullary thyroid carcinoma. J Gene Med 7, 179-188 (2005).
3. Alemany, R. & Curiel, D.T. CAR-binding ablation does not change biodistribution and toxicity of adenoviral vectors. Gene Ther 8, 1347-1353 (2001).
4. Putzer, B.M., Stiewe, T., Parssanedjad, K., Rega, S. & Esche, H. E1A is sufficient by itself to induce apoptosis independent of p53 and other adenoviral gene products. Cell Death Differ 7, 177-188 (2000).
5. Yamaguchi, M., Saito, H., Suzuki, M. & Mori, K. Visualization of neurogenesis in the central nervous system using nestin promoter-GFP transgenic mice. Neuroreport 11, 1991-1996 (2000).
6. Franklin, K.B.J., and Paxinos, G. The mouse brain in stereotaxic coordinates. Academic Press, San Diego (1997).

## Claims

1. Virus particle comprising a DNA sequence and at least one capsid protein, **characterized in that** the capsid protein is linked to a peptide selectively binding to neural precursor cells, wherein the viral vector is derived from a lentivirus, a retrovirus, an adenovirus, an adeno-associated virus, an Epstein-Barr virus, a herpes virus, an attenuated HIV or a vaccinia virus, and the neural precursor cells have the ability to self-renew and express the cell marker nestin, further **characterized in that** the peptide which selectively binds to neural precursor cells comprises at least one of the following amino acid sequences TPIQDYT (SEQ ID NO:1), QTRFLLH (SEQ ID NO:2), VPTQSSG (SEQ ID NO:3) or QTFTRMY (SEQ ID NO:4).

2. Virus particle according claim 1, **characterized in that** the peptide which selectively binds to neural precursor cells is covalently linked to a capsid protein.

3. Virus particle according to any of the preceding claims, **characterized in that** the peptide which selectively binds to neural precursor cells consists of 5 to 15 amino acids.

4. Virus particle according to any of the preceding claims, **characterized in that** the peptide which selectively binds to neural precursor cells consists of at least one of the following amino acid sequences TPIQDYT (SEQ ID NO:1), QTRFLLH (SEQ ID NO:2), VPTQSSG (SEQ ID NO:3) or QTFTRMY (SEQ ID NO:4).

5. Virus particle according to any of the preceding claims, **characterized in that** the DNA sequence comprises a gene encoding a therapeutic protein or a sequence encoding a small interfering RNA (siRNA).

6. Virus particle according to any of the preceding claims **characterized in that** the virus particle is an adenovirus particle, comprising a DNA sequence and at least one capsid protein, **characterized in that** the capsid protein is linked to a peptide consisting of 5 to 15 amino acids which peptide selectively binds to neural precursor cells, wherein the DNA sequence comprises a gene encoding a therapeutic protein or a sequence encoding a small interfering RNA (siRNA).

7. Virus particle according to any of the preceding claims, **characterized in that** the DNA sequence comprises a gene encoding a protein which promotes lineage-specific differentiation of neural precursor cells.

8. Virus particle according to any of the preceding claims, **characterized in that** the DNA sequence comprises a gene encoding a growth factor, such as EGF or FGF, a neurotrophic factor, such as BDNF, neurotrophin NT-3, NT-4, CTNF, GDNF, leukemia inhibitory factor or LIF, cardiotrophin-1 as well as oncostatin M, Neprilysin, GDNF, glutamic acid decarboxylase GAD-65/GAD-67 or the adenosine kinase inhibitor.

9. Virus particle according to any of the preceding claims, **characterized in that** the DNA sequence encodes a siRNA against BACE1, apolipoprotein E (ApoE) or GDNF.

10. Virus particle according to any of the preceding claims, **characterized in that** the peptide selectively binding to neural precursor cells reduces the binding of
(a) a phage expressing a peptide of the same sequence; or
(b) a virus comprising a peptide of the same sequence linked to a virus capsid protein or integrated into the virus capsid protein;
to neural precursor cells by a factor of at least 25%, preferably by at least 35% or by about 50%, if the isolated peptide selectively binding to neural precursor cells is present in the same concentration under conditions, where a peptide of unrelated sequence reduces the binding of the selectively binding peptide by less than 10%.

11. Virus particle according to any of the preceding claims, **characterized in that** peptide selectively binding to NPC binds differentiated neural cells with less than 50%, preferably less than 75% or less than 85% of the affinity of binding NPC.

12. Virus particle according to any of the preceding claims, **characterized in that** binding affinity of the peptide selectively binding to neural precursor cells is determined by incubating phages expressing the peptide on their surface with the respective cell line, washing off unbound material, washing off bound phages and determining the number of the bound phages.

13. Viral vector, comprising a DNA sequence encoding fusion polypeptide of a viral capsid protein and a peptide selectively binding to neural precursor cells, wherein the viral vector is derived from a lentivirus, a retrovirus, an adenovirus, an adeno-associated virus, an Epstein-Barr virus, a herpes virus, an attenuated HIV or a vaccinia virus, and the neural precursor cells have the ability to self-renew and express the cell marker nestin, further **characterized in that** the peptide which selectively binds to neural precursor cells comprises at least one of the following amino acid sequences TPIQDYT (SEQ ID NO:1), QTRFLLH (SEQ ID NO:2), VPTQSSG (SEQ ID NO:3) or QTFTRMY (SEQ ID NO:4).

14. Peptide comprising the amino acid sequence TPIQDYT (SEQ ID NO:1), QTRFLLH (SEQ ID NO:2), VPTQSSG (SEQ ID NO:3) or QTFTRMY (SEQ ID NO:4), wherein the peptide consists of 5 to 15 amino acids.

15. Pharmaceutical composition comprising a virus particle or a viral vector according to one of claims 1 to 13.

16. Pharmaceutical composition according to claim 15 further comprising a pharmaceutically acceptable carrier.

17. Pharmaceutical composition according to claim 15 or 16, for use in treating a neurodegenerative disease, such as Alzheimer Disease or Parkinson Disease, ischemia, failing adult hippocampal neurogenesis, dementia, depression, temporal lobe epilepsy, Multiple Sclerosis (MS), Amyotrophic Lateral Sclerosis (ALS) or cancer.

18. Pharmaceutical composition according to claim 17, wherein neurodegenerative disease is Alzheimer Disease or Parkinson Disease.

## Patentansprüche

1. Viruspartikel umfassend eine DNA Sequenz und mindestens ein Kapsidprotein, **dadurch gekennzeichnet, dass** das Kapsidprotein mit einem Peptid verknüpft ist, welches selektiv an neurale Vorläuferzellen bindet, wobei der virale Vektor von einem Lentivirus, einem Retrovirus, einem Adenovirus, einem Adeno-assoziirten Virus, einem Epstein-Barr Virus, einem Herpesvirus, einem attenuierten HIV or einem Vacciniavirus abstammt, und die neurale Vorläuferzelle die Fähigkeit zur Selbsterneuerung und zur Expression des Zellmarkers Nestin aufweist, ferner **dadurch gekennzeichnet, dass** das Peptid, welches selektiv an die neurale Vorläuferzelle bindet, mindestens eine der folgenden Aminosäuresequenzen umfasst TPIQDYT (SEQ ID NR:1), QTRFLLH (SEQ ID NR:2), VPTQSSG (SEQ ID NR:3) oder QTFTRMY (SEQ ID NR:4).

2. Viruspartikel gemäß Anspruch 1, **dadurch gekennzeichnet dass** das Peptid, welches selektive an neurale Vorläuferzellen bindet, kovalent mit dem Kapsidprotein verknüpft ist.

3. Viruspartikel gemäß einem der vorstehenden Ansprüche, **dadurch gekennzeichnet, dass** das Peptid, welches selektiv an neurale Vorläuferzellen bindet, aus 5 bis 15 Aminosäuren besteht.

4. Viruspartikel gemäß einem der vorstehenden Ansprüche, **dadurch gekennzeichnet, dass** das Peptid, welches selektiv an neurale Vorläuferzellen bindet, aus mindestens einer der folgenden Aminosäuresequenzen besteht TPIQDYT (SEQ ID NR:1), QTRFLLH (SEQ ID NR:2), VPTQSSG (SEQ ID NR:3) oder QTFTRMY (SEQ ID NR:4).

5. Viruspartikel gemäß einem der vorstehenden Ansprüche, **dadurch gekennzeichnet, dass** die DNA Sequenz ein Gen umfasst, dass für ein therapeutisches Protein oder für eine small interfering RNA (siRNA) kodiert.

6. Viruspartikel gemäß einem der vorstehenden Ansprüche, **dadurch gekennzeichnet, dass** das Viruspartikel ein Adenoviruspartikel ist, welches eine DNA Sequenz und mindestens ein Kapsidprotein umfasst, ferner **dadurch gekennzeichnet, dass** das Kapsidprotein mit einem Peptid bestehend aus 5 bis 15 Aminosäuren verknüpft ist, wobei das Peptid selektiv an neurale Vorläuferzellen bindet, wobei die DNA Sequenz ein Gen, dass für ein therapeutisches Protein kodiert, oder eine Sequenz umfasst, die für eine small interfering RNA (siRNA) kodiert.

7. Viruspartikel gemäß einem der vorstehenden Ansprüche, **dadurch gekennzeichnet, dass** die DNA Sequenz ein Gen umfasst, dass für ein Protein kodiert, welches Zelltypspezifische Differenzierung neuraler Vorläuferzellen fördert.

8. Viruspartikel gemäß einem der vorstehenden Ansprüche, **dadurch gekennzeichnet, dass** die DNA Sequenz ein Gen umfasst, dass für einen Wachstumsfaktor, wie EGF oder FGF, einen neurotrophen Faktor, wie BDNF, Neurotrophin NT-3, NT-4, CTNF, GDNF, einen Leukämie-hemmenden Faktor oder LIF, Cardiotrophin-1 sowie Oncostatin M, Neprilysin, GDNF, Glutamat-Decarboxylase GAD-65/GAD-67 oder einen Adenosinkinase-Inhibitor kodiert.

9. Viruspartikel gemäß einem der vorstehenden Ansprüche, **dadurch gekennzeichnet, dass** die DNA Sequenz für eine siRNA gegen BACE1, Apolipoprotein E (ApoE) oder GDNF kodiert.

10. Viruspartikel gemäß einem der vorstehenden Ansprüche, **dadurch gekennzeichnet, dass** das Peptid, welches selektiv an neurale Vorläuferzellen bindet, die Bindung:
(a) eines Phagen, der ein Peptid derselben Sequenz exprimiert; oder
(b) eines Virus, der ein Peptid derselben Sequenz umfasst, welches mit einem Viruskapsidprotein verknüpft ist oder in das Viruskapsidprotein integriert ist;
an neurale Vorläuferzellen um einen Faktor von mindestens 25%, vorzugsweise mindestens 35% oder etwa 50% reduziert, wenn das isolierte Peptid, das selektiv an neurale Vorläuferzellen bindet, in der gleichen Konzentration und unter Bedingungen vorliegt, bei denen ein Peptid ohne Sequenzidentität die Bindung des selektiv bindenden Peptids um weniger als 10% reduziert.

11. Viruspartikel gemäß einem der vorstehenden Ansprüche, **dadurch gekennzeichnet, dass** das Peptid, welches selektiv an neurale Vorläuferzellen bindet, differenzierte neurale Zellen mit einer Affinität von weniger als 50%, vorzugsweise weniger als 75% oder weniger als 85% der Affinität für neurale Vorläuferzellen bindet.

12. Viruspartikel gemäß einem der vorstehenden Ansprüche, **dadurch gekennzeichnet, dass** die Bindungsaffinität des Peptids, welches selektiv an neurale Vorläuferzellen bindet, durch Inkubation von Phagen und entsprechenden Zelllinien bestimmt wird, wobei die Phagen das Peptid auf ihrer Oberfläche exprimieren, nicht-gebundenes Material abgewaschen wird und die Anzahl der gebundenen Phagen bestimmt wird.

13. Viraler Vektor, umfassend eine DNA Sequenz, die für ein Fusionspolypeptid aus einem viralen Kapsidprotein und einem Peptid kodiert, welches selektiv an neurale Vorläuferzellen bindet, wobei der virale Vektor von einem Lentivirus, einem Retrovirus, einem Adenovirus, einem Adeno-assoziirten Virus, einem Epstein-Barr Virus, einem Herpesvirus, einem attenuierten HIV oder einem Vacciniavirus abstammt, und die neuralen Vorläuferzellen die Fähigkeit zur Selbsterneuerung und zur Expression des Zellmarkers Nestin aufweist, ferner **dadurch gekennzeichnet, dass** das Peptid, welches selektiv an die neurale Vorläuferzelle bindet, mindestens eine der folgenden Aminosäuresequenzen umfasst TPIQDYT (SEQ ID NR:1), QTRFLLH (SEQ ID NR:2), VPTQSSG (SEQ ID NR:3) oder QTFTRMY (SEQ ID NR:4).

14. Peptid umfassend die Aminosäuresequenzen TPIQDYT (SEQ ID NR:1), QTRFLLH (SEQ ID NR:2), VPTQSSG (SEQ ID NR:3) oder QTFTRMY (SEQ ID NR:4), wobei das Peptid aus 5 bis 15 Aminosäuren besteht.

15. Pharmazeutische Zusammensetzung, umfassend einen Viruspartikel oder einen viralen Vektor gemäß einem der Ansprüche 1 bis 13.

16. Pharmazeutische Zusammensetzung gemäß Anspruch 15, die ferner einen pharmazeutisch verträglichen Träger umfasst.

17. Pharmazeutische Zusammensetzung gemäß irgendeinem der Ansprüche 15 oder 16, zur Verwendung bei der Behandlung einer neurodegenerativen Erkrankung, wie Alzheimer, Parkinson, Ischämie, mangelhafte adulte Hypokampale Neurogenese, Demenz, Depression, Temporallappeneplilepsie, Multiple Sklerose (MS), Amyotrophe Laterale Sklerose (ALS) oder Krebs.

18. Pharmazeutische Zusammensetzung gemäß Anspruch 17, bei der die neurodegenerative Erkrankung Alzheimer oder Parkinson ist.

## Revendications

1. Particule virale comprenant une séquence d'ADN et au moins une protéine capsidique, **caractérisée en ce que** la protéine capsidique est liée à un peptide se liant sélectivement à des cellules précurseurs neurales, le virus étant issu d'un lentivinis, d'un rétrovirus, d'un adénovinis, d'un virus associé aux adénovinis, d'un virus Epstein-Barr, d'un herpes virus, d'un VIH atténué ou d'un virus de la vaccine, et les cellules précurseurs neurales ayant l'aptitude à s'auto-renouveler et à exprimer le marqueur cellulaire nestine, en outre **caractérisée en ce que** le peptide qui se lie sélectivement à des cellules précurseurs neurales comprend au moins l'une des séquences d'acides aminés suivantes TPIQDYT (SEQ ID N° 1), QTRFLLH (SEQ ID N° 2), VPTQSSG (SEQ ID N° 3) ou QTFTRMY (SEQ ID N° 4).

2. Particule virale selon la revendication 1, **caractérisée en ce que** le peptide qui se lie sélectivement aux cellules précurseurs neurales est lié par covalence à une protéine capsidique.

3. Particule virale selon l'une quelconque des revendications précédentes, **caractérisée en ce que** le peptide qui se lie sélectivement aux cellules précurseurs neurales consiste en 5 à 15 acides aminés.

4. Particule virale selon l'une quelconque des revendications précédentes, **caractérisée en ce que** le peptide qui se lie sélectivement aux cellules précurseurs neurales est constitué d'au moins l'une des séquences d'acides aminés suivantes TPIQDYT (SEQ ID N° 1), QTRFLLH (SEQ ID N° 2), VPTQSSG (SEQ ID N° 3) ou QTFTRMY (SEQ ID N° 4).

5. Particule virale selon l'une quelconque des revendications précédentes, **caractérisée en ce que** la séquence d'ADN comprend un gène codant une protéine thérapeutique ou une séquence codant un petit ARN interférant (pARNi).

6. Particule virale selon l'une quelconque des revendications précédentes, **caractérisée en ce que** la particule virale est une particule d'adénovirus, comprenant une séquence d'ADN et au moins une protéine capsidique, **caractérisée en ce que** la protéine capsidique est liée à un peptide constitué de 5 à 15 acides aminés, lequel peptide se lie sélectivement à des cellules précurseurs neurales, la séquence d'ADN comprenant un gène codant une protéine thérapeutique ou une séquence codant une petit ARN interférant (pARNi).

7. Particule virale selon l'une quelconque des revendications précédentes, **caractérisée en ce que** la séquence d'ADN comprend un gène codant une protéine qui stimule la différenciation spécifique de lignée de cellules précurseurs neurales.

8. Particule virale selon l'une quelconque des revendications précédentes, **caractérisée en ce que** la séquence d'ADN comprend un gène codant un facteur de croissance, tel que l'EGF ou le FGF, ou un facteur neurotrophique, tel que le BDNF, la neurotrophine NT-3, NT-4, CTNF, GDNF, le facteur inhibiteur de leucémie ou LIF, la cardiotrophine 1 ainsi que l'oncostatine M, la néprilysine, le GDNF, l'acide glutamique décarboxylasee GAD-65/GAD-67 ou l'inhibiteur d'adénosine kinase.

9. Particule virale selon l'une quelconque des revendications précédentes, **caractérisée en ce que** la séquence d'ADN code un pARNi contre BACE1, l'apoliprotéine E (ApoE) ou GDNF.

10. Particule virale selon l'une quelconque des revendications précédentes, **caractérisée en ce que** le peptide se liant sélectivement aux cellules précurseurs neurales réduit la liaison
(a) d'un phage exprimant un peptide ayant la même séquence ; ou
(b) d'un virus comprenant un peptide ayant la même séquence, lié à une protéine de capside virale ou intégré dans la protéine de capside virale ;
à des cellules précurseurs neurales par un facteur d'au moins 25 %, de préférence d'au moins 35 % ou d'environ 50 %, si le peptide isolé se liant sélectivement à des cellules précurseurs neuronales est présent à la même concentration, dans des conditions dans lesquelles un peptide à séquence non apparentée réduit de moins de 10 % la liaison du peptide se liant sélectivement.

11. Particule virale selon l'une quelconque des revendications précédentes, **caractérisée en ce que** le peptide se liant sélectivement à des CPN [cellules précurseurs neurales] se lie à des cellules neurales différenciées avec moins de 50 %, de préférence moins de 75 % ou moins de 85 % de l'affinité de liaison aux CPN.

12. Particule virale selon l'une quelconque des revendications précédentes, **caractérisée en ce que** l'affinité de liaison du peptide se liant sélectivement à des cellules précurseurs neurales est déterminée par incubation de phages exprimant le peptide à leur surface avec la lignée cellulaire respective, élimination par lavage de la substance non liée, séparation par lavage des phages liés et détermination du nombre des phages liés.

13. Vecteur viral, comprenant une séquence d'ADN codant un polypeptide de fusion d'une protéine de capside virale et d'un peptide se liant sélectivement aux cellules précurseurs neurales, le vecteur viral étant issu d'un lentivinis, d'un rétrovirus, d'un adénovinis, d'un virus associé aux adénovinis, d'un virus Epstein-Barr, d'un herpes virus, d'un VIH atténué ou d'un virus de la vaccine, et les cellules précurseurs neurales ayant l'aptitude à s'auto-renouveler et à exprimer le marqueur cellulaire nestine, en outre **caractérisé en ce que** le peptide qui se lie sélectivement à des cellules précurseurs neurales comprend au moins l'une des séquences d'acides aminés suivantes TPIQDYT (SEQ ID N° 1), QTRFLLH (SEQ ID N° 2), VPTQSSG (SEQ ID N° 3) ou QTFTRMY (SEQ ID N° 4).

14. Peptide comprenant la séquence d'acides aminés TPIQDYT (SEQ ID N° 1), QTRFLLH (SEQ ID N° 2), VPTQSSG (SEQ ID N° 3) ou QTFTRMY (SEQ ID N° 4), le peptide étant constitué de 5 à 15 acides aminés.

15. Composition pharmaceutique comprenant une particule virale ou un vecteur viral selon l'une quelconque des revendications 1 à 13.

16. Composition pharmaceutique selon la revendication 15, comprenant en outre un véhicule pharmaceutiquement acceptable.

17. Composition pharmaceutique selon la revendication 15 ou 16, pour utilisation dans le traitement d'une maladie neurodégénérative, telle que la maladie d'Alzheimer ou la maladie de Parkinson, l'ischémie, la neurogenèse déficiente de l'hippocampe de l'adulte, la démence, la dépression, l'épilepsie du lobe temporal, la sclérose multiple (SM), la sclérose latérale amyotrophique (SLA) ou le cancer.

18. Composition pharmaceutique selon la revendication 17, la maladie neurodégénérative étant la maladie d'Alzheimer ou la maladie de Parkinson.


### SEQUENCE LISTING

<110> University of Rostock
<120> Selective Targeting of Viruses to Neural Precursor Cells
<130> P 73687
<160> 71
<170> PatentIn version 3.4
<210> 1
   <211> 7
   <212> PRT
   <213> Artificial
<220>
   <223> oligopeptide
<400> 1
   Thr Pro Ile Gln Asp Tyr Thr
   1               5
<210> 2
   <211> 7
   <212> PRT
   <213> Artificial
<220>
   <223> oligopeptide
<400> 2
   Gln Thr Arg Phe Leu Leu His
   1               5
<210> 3
   <211> 7
   <212> PRT
   <213> Artificial
<220>
   <223> oligopeptide
<400> 3
   Val Pro Thr Gln Ser Ser Gly
   1 5
<210> 4
   <211> 7
   <212> PRT
   <213> Artificial
<220>
   <223> oligopeptide
<400> 4
   Gln Thr Phe Thr Arg Met Tyr
   1 5
<210> 5
   <211> 7
   <212> PRT
   <213> Artificial
<220>
   <223> oligopeptide
<400> 5
   His Thr Phe Glu Pro Gly Val
   1 5